# EUROPEAN PATENT APPLICATION

(11) **EP 2 179 990 A1**
(43) Date of publication of application: **28.04.2010**
(21) Application number: 08356137.3
(22) Date of filing: 24.10.2008
(51) Int. Cl.: C07D 215/20, A01N 43/42

(54) **Fungicide oxyalkylamide derivatives**

(71) Applicant: Bayer CropScience SA, 69009 Lyon (FR)
(72) Inventor: Coqueron, Pierre-Yves, 69003 Lyon (FR); Ort, Oswald, 51375 Leverkusen (DE); Rahn, Nicola, 40789 Monheim am Rhein (DE); Toquin, Valérie, 69270 St Romain au Mont d'Or (FR); Wachendorff-Neumann, Ulrike, 56566 Neuwied (DE)
(74) Representative: Balmefrezol, Ludovic Francis Pierre

(57) **Abstract**

The present invention relates to fungicide oxyalkylamide derivatives of formula (I), their process of preparation, their use as fungicides, particularly in the form of fungicidal compositions and methods for the control of phytopathogenic fungi of plants using these compounds or their compositions. 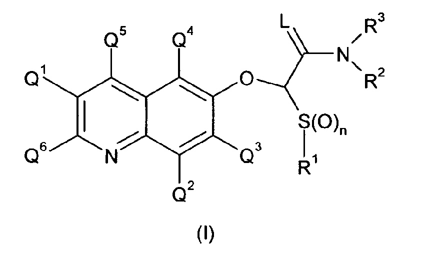 wherein Q¹ to Q⁶, R¹ to R³, n and L represent various substituents.

## Description

The present invention relates to oxyalkylamide derivatives, their process of preparation, their use as fungicide active agents, particularly in the form of fungicide compositions, and methods for the control of phytopathogenic fungi, notably of plants, using these compounds or compositions.

In international patent application WO 2008/028624, there are disclosed certain sulphur-substituted quinolyloxyalkanoïc acid amides derivatives of the following chemical structure: 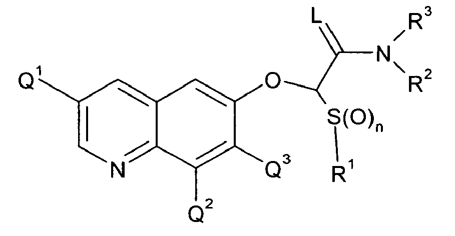 wherein R¹ represents an alkyl group, an alkenyl group, an alkynyl group or a cycloakyl group.
The compounds disclosed in this document do not prove to provide a comparable utility than the compounds according to the invention.

It is always of high-interest in agriculture to use novel pesticide compounds in order to avoid or to control the development of resistant strains to the active ingredients. It is also of high-interest to use novel compounds being more active than those already known, with the aim of decreasing the amounts of active compound to be used, whilst at the same time maintaining effectiveness at least equivalent to the already known compounds. We have now found a new family of compounds which possess the above mentioned effects or advantages.

Accordingly, the present invention provides an oxyalkylamide derivative of formula (I) 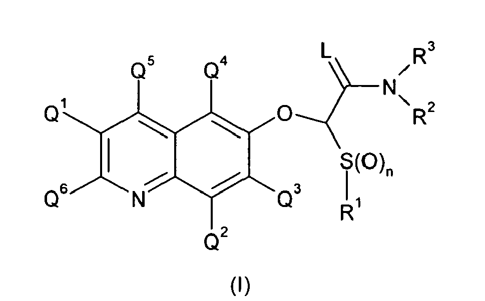 wherein
- Q¹ and Q² independently represent a hydrogen atom, a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶-sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
- Q³ represents a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-□⁶-sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
- Q⁴, Q⁵ and Q⁶ independently represent a hydrogen atom, a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silylC₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S; provided that at least one of Q⁴, Q⁵ or Q⁶ does not represent a hydrogen atom;
- R¹ represents a hydrogen atom, substituted or non-substituted C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted aryl, substituted or non-substituted aryl-[C₁-C₈]-alkyl, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
- R² represents a hydrogen atom, a hydroxy group, a cyano group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkoxy, substituted or non-substituted benzyloxy:

- R³ represents -(CR^{a}R^{b})ₚ(CR^{c}R^{d})_{q}(X)ᵣ(CR^{e}R^{f})ₛR⁴, wherein
   ○ R^{a}, R^{b}, R^{c}, R^{d}, R^{e} and R^{f}, independently represent a hydrogen atom, substituted or non-substituted C₁-C₈-alkyl, a halogen atom, a cyano group, a hydroxy group, substituted or non-substituted C₁-C₈-alkoxy or substituted or non-substituted C₁-C₈-alkoxycarbonyl, or
   ○ R^{a}R^{b}, R^{c}R^{d} or R^{e}R^{f} form a 3- to 8-membered carbocyclic or heterocyclic ring comprising a heteroatom selected from sulfur, oxygen and NR⁰ wherein R⁰ represents a hydrogen atom or substituted or non-substituted C₁-C₈-alkyl;
   ○ X represents (CO), (CO)O, O(CO), 0, S(O)ₜ, wherein t is 0, 1 or 2; or X represents NH, substituted or non-substituted C₁-C₈-alkylamino ;
   ○ p, r and s, independently represent 0 or 1 ;
   ○ q represents 0, 1 or 2 ;
   ○ R⁴ represents a halogen atom, substituted or non-substituted C₁-C₈-alkyl, substituted or non-substituted C₁-C₈-alkenyl, -CR^{UU}=NR^{W} wherein R^{uu} represents a hydrogen atom or substituted or non-substituted C₁-C₈-alkyl and R^{w} represents a hydroxy group or substituted or non-substituted C₁-C₈-alkoxy, substituted or non-substituted aryloxy or substituted or non-substituted heteroaryloxy or R⁴ represents CH₂-C≡C-R⁵ wherein R⁵ represents a hydrogen atom, substituted or non-substituted C₁-C₈-alkyl, substituted or non-substituted C₃-C₈-cycloalkyl, substituted or non-substituted aryl, substituted or non-substituted aryl-[C₁-C₈]-alkyl, substituted or non-substituted tri(C₁-C₈-alkyl)-silyl; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S, or R⁴ represents substituted or non-substituted C₃-C₈-cycloalkyl, substituted or non-substituted C₃-C₈-cycloalkenyl, substituted or non-substituted aryl, saturated or unsaturated 4-, 5-, 6- or 7-membered heterocyclyl comprising up to 4 heteroatoms selected in the list consisting of N, O, S; or
- when R³ represents -(CR^{a}R^{b})ₚ(CR^{c}R^{d})_{q}(X)ᵣ(CR^{e}R^{f})ₛR⁴, R² and R³ form a 5- or 6-membered ring optionally substituted with a halogen atom, C₁-C₈-alkyl, mono- or di-C₁-C₈-alkylaminocarbonyl, and optionally containing a heteroatom selected from sulphur, oxygen and NR⁰⁰, wherein R⁰⁰ represents C₁-C₈-alkyl, C₁-C₈-halogenoalkyl having 1 to 5 halogen atoms, C₁-C₈-alkoxy, cyano, substituted or non-substituted aryl; or
- R² and R³ form an optionally substituted 6,6-membered bicycle; or
- R³ represents -(CR³⁰R⁴⁰)C≡CR⁵⁰, wherein
   ○ R³⁰ and R⁴⁰, independently represent a hydrogen atom, substituted or non-substituted C₁-C₈-alkyl, substituted or non-substituted C₁-C₈-halogenoalkyl having 1 to 5 halogen atoms, a C₂-C₈-alkenyl, substituted or non-substituted C₂-C₈-alkynyl, or
   ○ R³⁰ and R⁴⁰ join with the carbon atom to which they are attached to form a substituted or non-substituted 3- to 6-membered carbocyclic or heterocyclic ring containing a heteroatom selected from sulfur, oxygen or NR⁰⁰⁰ wherein R⁰⁰⁰ represents a hydrogen atom, C₁-C₈-alkyl;
   ○ R⁵⁰ represents a hydrogen atom, substituted or non-substituted C₁-C₈-alkyl, substituted or non-substituted C₃-C₈-cycloalkyl" substituted or non-substituted aryl, saturated or unsaturated 4-, 5-, 6- or 7-membered heterocyclyl comprising up to 4 heteroatoms selected in the list consisting of N, O, S,
- n is 0,1 or 2 ;
- L represents a sulfur atom or an oxygen atom ; as well as salts, N-oxides, metallic complexes and metalloidic complexes thereof.

Any of the compounds according to the invention can exist as one or more stereoisomers depending on the number of stereogenic units (as defined by the IUPAC rules) in the compound. The invention thus relates equally to all the stereoisomers, and to the mixtures of all the possible stereoisomers, in all proportions. The stereoisomers can be separated according to the methods which are known per se by the man ordinary skilled in the art.

According to the invention, the following generic terms are generally used with the following meanings:
- halogen means fluorine, chlorine, bromine or iodine;
- heteroatom can be nitrogen, oxygen or sulphur ;
- unless indicated otherwise, a group or a substituent that is substituted according to the invention can be substituted by one or more of the following groups or atoms: a halogen atom, a nitro group, a hydroxy group, a cyano group, an amino group, a sulphenyl group, a pentafluoro-□⁶-sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a (hydroxyimino)-C₁-C₆-alkyl group, a C₁-C₈-alkyl, a tri(C₁-C₈-alkyl)silyl-C₁-C₈-alkyl, C₁-C₈-cycloalkyl, tri(C₁-C₈alkyl)silyl-C₁-C₈ycloalkyl, a C₁-C₈-halogenoalkyl having 1 to 5 halogen atoms, a C₁-C₈-halogenocycloalkyl having 1 to 5 halogen atoms, a C₂-C₈-alkenyl, a C₂-C₈-alkynyl, a C₂-C₈-alkenyloxy, a C₂-C₈-alkynyloxy, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulphenyl, a C₁-C₈-halogenoalkylsulphenyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, substituted or non-substituted C₁-C₈-alkoxycarbonylamino, substituted or non-substituted C₁-C₈-halogenoalkoxycarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₈-alkylaminosulfamoyl, a di-C₁-C₈-alkylaminosulfamoyl, a (C₁-C₆-alkoxyimino)-C₁-C₆-alkyl, a (C₁-C₆-alkenyloxyimino)-C₁-C₆-alkyl, a (C₁-C₆-alkynyloxyimino)-C₁-C₆-alkyl, (benzyloxyimino)-C₁-C₆-alkyl, C₁-C₈-alkoxyalkyl, C₁-C₈-halogenoalkoxyalkyl having 1 to 5 halogen atoms, benzyloxy, benzylsulphenyl, benzylamino, phenoxy, phenylsulphenyl, or phenylamino ; • the term "aryl" means phenyl or naphthyl.

Preferred compounds of formula (I) according to the invention are those wherein Q¹ and Q² independently represent a hydrogen atom, a halogen atom, a cyano group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₃-C₈-alkynyloxy, substituted or non-substituted phenoxy, substituted or non-substituted aryl, substituted or non-substituted aryl-[C₁-C₈]-alkyl, substituted or non-substituted tri(C₁-C₈-alkyl)-silyloxy, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-,5-,6- or 7-membered heterocyclyl-[C₁-C₈]-alkyl comprising up to 4 heteroatoms selected in the list consisting of N, O, S.

More preferred compounds of formula (I) according to the invention are those wherein Q¹ and Q² independently represent a hydrogen atom, a halogen atom, substituted or non-substituted C₁-C₈-alkyl.

Other preferred compounds of formula (I) according to the invention are those wherein Q³ represents a halogen atom, a cyano group, substituted or non-substituted C₁-C₈-alkyl, substituted or non-substituted tri(C₁-C₈-alkyl)silyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy.

Other more preferred compounds of formula (I) according to the invention are those wherein Q³ represents a halogen atom, substituted or non-substituted C₁-C₈-alkyl.

Other preferred compounds of formula (I) according to the invention are those wherein Q⁴' Q⁵ and Q⁶ independently represent a hydrogen atom, a halogen atom, a cyano group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₃-C₈-alkynyloxy, substituted or non-substituted phenoxy, substituted or non-substituted aryl, substituted or non-substituted aryl-[C₁-C₈]-alkyl, substituted or non-substituted tri(C₁-C₈-alkyl)-silyloxy, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S.

Other preferred compounds of formula (I) according to the invention are those wherein R¹ represents a hydrogen atom, a hydroxy group, a substituted or non-substituted carbaldehyde O-(C₁-C₈-alkyl)oxime, a formyloxy group, substituted or non-substituted C₁-C₈-alkoxyamino group, substituted or non-substituted N-C₁-C₈-alkyl-(C₁-C₈-alkoxy)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkoxy, substituted or non-substituted C₁-C₈-halogenoalkoxy having 1 to 5 halogen atoms, substituted or non-substituted C₂-C₈-alkenyloxy, substituted or non-substituted C₃-C₈-alkynyloxy, substituted or non-substituted C₁-C₈-alkylcarbonyloxy, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted phenoxy, substituted or non-substituted phenylsulphenyl, substituted or non-substituted aryl, substituted or non-substituted aryl-[C₁-C₈]-alkyl, substituted or non-substituted tri(C₁-C₈-alkyl)-silyloxy.

Other more preferred compounds of formula (I) according to the invention are those wherein R¹ represents a substituted or non-substituted C₁-C₈-alkyl, substituted or non-substituted C₃-C₈-cycloalkyl, substituted or non-substituted C₁-C₈-halogenoalkyl having 1 to 5 halogen atoms, a C₂-C₈-alkenyl, substituted or non-substituted C₂-C₈-alkynyl, substituted or non-substituted aryl, substituted or non-substituted aryl-[C₁-C₈]-alkyl.

Even more preferred compounds of formula (I) according to the invention are those wherein R¹ represents a substituted or non-substituted C₁-C₈-alkyl, substituted or non-substituted C₃-C₈-cycloalkyl, a C₂-C₈-alkenyl, substituted or non-substituted C₂-C₈-alkynyl.

Other preferred compounds of formula (I) according to the invention are those wherein R² represents a hydrogen atom, substituted or non-substituted C₁-C₈-alkyl, substituted or non-substituted C₁-C₈-cycloalkyl.

Other preferred compounds of formula (I) according to the invention are those wherein L represents an oxygen atom.

The above mentioned preferences with regard to the substituents of the compounds of formula (I) according to the invention can be combined in various manners. These combinations of preferred features thus provide sub-classes of compounds according to the invention. Examples of such sub-classes of preferred compounds according to the invention can combine:
- preferred features of Q¹ with preferred features of Q² to Q⁶, R¹ to R³, n and L;
- preferred features of Q² with preferred features of Q¹, Q³ to Q⁶, R¹ to R³, n and L;
- preferred features of Q³ with preferred features of Q¹, Q², Q⁴ to Q⁶, R⁷ to R³, n and L;
- preferred features of Q⁴ with preferred features of Q¹ to Q³, Q⁵, Q⁶, R¹ to R³, n and L;
- preferred features of Q⁵ with preferred features of Q¹ to Q⁴, Q⁶, R¹ to R³, n and L;
- preferred features of Q⁶ with preferred features of Q¹ to Q⁵, R¹ to R³, n and L;
- preferred features of R¹ with preferred features of Q¹ to Q⁶, R² and R³, n and L;
- preferred features of R² with preferred features of Q¹ to Q⁶, R¹, R³ and L;
- preferred features of R³ with preferred features of Q¹ to Q⁶, R¹, R² and L;
- preferred features of n with preferred features of Q¹ to Q⁶ and R¹ to R³ and L;
- preferred features of L with preferred features of Q¹ to Q⁶, R¹ to R³ and n.

In these combinations of preferred features of the substituents of the compounds according to the invention, the said preferred features can also be selected among the more preferred features of each of Q¹ to Q⁶, R¹ to R³, n and L, so as to form most preferred subclasses of compounds according to the invention.

The present invention also relates to a process for the preparation of compounds of formula (I).

Thus, according to a further aspect of the present invention, there is a provided process P1 for the preparation of compounds of formula (Ia), as herein-defined and illustrated by the following reaction scheme: 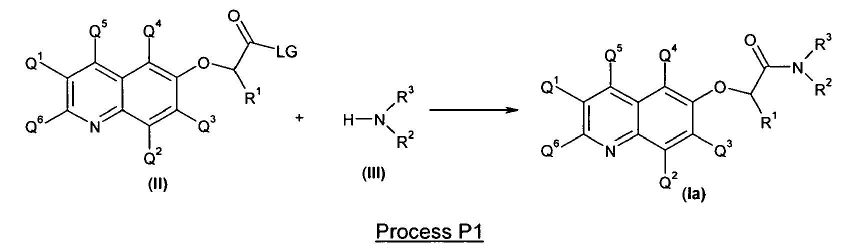 by reacting a compound of formula (II), wherein LG represents a leaving group, Q¹, Q², Q³, Q⁴, Q⁵, Q⁶ and R¹, being as herein-defined with a compound of formula (III) wherein R², R³ are as herein-defined; optionally in the presence of a base such as an inorganic or an organic base; notably an alkaline earth metal or an alkali metal hydride, hydroxide, amide, alcoholate, acetate, carbonate or hydrogen carbonate, such as sodium hydride, sodium amide, lithiium diisopropylamide, sodium methanolate, sodium ethanolate, potassium tert-butanolate, sodium acetate, potassium acetate, calcium acetate, sodium hydroxide, potassium hydroxide, sodium carbonate, potassium carbonate, potassium bicarbonate, sodium bicarbonate, cesium carbonate or ammonium carbonate; and also tertiary amines, such as trimethylamine, triethylamine (TEA), tributylamine, N,N-dimethylaniline, N,N-dimethyl-benzylamine, N,N-diisopropyl-ethylamine (DIPEA), pyridine, N-methylpiperidine, N-methylmorpholine, N,N-dimethylaminopyridine, diazabicyclooctane (DABCO), diazabicyclononene (DBN) or diazabicycloundecene (DBU), optionally in the presence of an acid such as a Lewis acid; notably metal or metalloïd halides such as aluminium trichloride, zinc dichloride, magnesium bromide, boron tribromide; or such as a Brönstedt acid; notably a mineral acid such as sulphuric acid, chlorhydric acid, ammonium chloride, phosphoric acid, or an organic acid, such as acetic acid, para-toluenesulphonic acid, optionally in the presence of a condensing agent such as acid halide former, notably phosgene, phosphorous tribromide, phosphorous trichloride, phosphorous pentachloride, phosphorous trichloride oxide or thionyl chloride; such as an anhydride former, notably ethyl chloroformate, methyl chloroformate, isopropyl chloroformate, isobutyl chloroformate, 2,2-dimethylpropionyl chloride or methanesulfonyl chloride; notably carbodiimides, such as N,N'-dicyclohexylcarbodiimide (DCC) or such as other customary condensing agents, notably phosphorous pentoxide, polyphosphoric acid, N,N'-carbonyldiimidazole, 2-ethoxy-N-ethoxycarbonyl-1,2-dihydroquinoline (EEDQ), triphenylphosphine/tetrachloromethane or bromo-tripyrrolidinophosphonium-hexafluorophosphate, optionally in the presence of a catalyst notably a transition metal catalyst, such as palladium salts or complexes for example palladium (II) chloride, palladium (II) acetate, tetrakis-(triphenylphosphine) palladium(0), bis-(triphenylphosphine) palladium dichloride (II), tris(dibenzylideneacetone) dipalladium(0), bis(dibenzylideneacetone) palladium(0), or 1,1'-bis(diphenylphosphino)ferrocene-palladium (II) chloride. As an alternative the palladium complex is directly generated in the reaction mixture by separately adding to the reaction mixture a palladium salt and a complex ligand such as a phosphine, for example triethylphosphine, tri-tert-butylphosphine, tricyclohexylphosphine, 2-(dicyclohexylphosphine)biphenyl, 2-(di-tert-butylphosphin)biphenyl, 2-(dicyclohexylphosphine)-2'-(N,N-dimethylamino)-biphenyl, triphenylphosphine, tris-(o-tolyl)phosphine, sodium 3-(diphenylphosphino)benzolsulfonate, tris-2-(methoxyphenyl)phosphine, 2,2'-bis-(diphenylphosphine)-1,1'-binaphthyl, 1,4-bis-(diphenylphosphine)butane, 1,2-bis-(diphenylphosphine)ethane, 1,4-bis-(dicyclohexylphosphine)butane, 1,2-bis-(dicyclohexylphosphine)ethane, 2-(dicyclohexylphosphine)-2'-(N,N-dimethylamino)-biphenyl, bis(diphenylphosphino)ferrocene, tris-(2,4-tert-butylphenyl)-phosphite, (R)-(-)-1-[(S)-2-(diphenylphosphino)ferrocenyl]ethyldi-tert-butylphosphine, (S)-(+)-1-[(R)-2-(diphenylphosphino)ferrocenyl]ethyldicyclohexylphosphine, (R)-(-)-1-[(S)-2-(diphenylphosphino)ferrocenyl]ethyldicyclohexylphosphine, (S)-(+)-1-[(R)-2-(diphenylphosphino)ferrocenyl]ethyldi-t-butylphosphine; in one or a different-pot conditions.

Suitable leaving groups LG can be selected in the list consisting of a halogen atom or other customary nucleofugal groups such as alcoolate, hydroxide or cyanide.

For the compounds of formula (Ia) according to the invention when L represents an oxygen atom, process P1 according to the invention can be completed by a further step comprising the additional modification of this group, notably by a reaction of thiocarbonylation in the presence of a thiocarbonylating agent such as 2,4-bis(4-methoxyphenyl)-1,3,2,4-dithiadiphosphetane 2,4-disulfide, phosphorus pentasulfide, sulphur to yield to a compound of formula (Ib), according to known methods. In such a case there is provided a process P2 according to the invention and such a process P2 can be illustrated by the following reaction scheme : 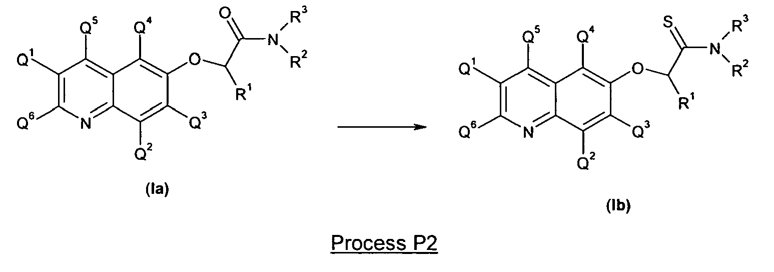 According to the invention, processes P1 and P2 can be performed if appropriate in the presence of a solvent or a base.

According to the invention, processes P1 can be performed if appropriate in the presence of a catalyst. Suitable catalyst can be chosen as being 4-dimethyl-aminopyridine, 1-hydroxybenzotriazole or dimethylformamide.
In case LG represents a hydroxy group, the process P2 according to the present invention can be performed in the presence of condensing agent. Suitable condensing agent can be chosen as being acid halide former, such as phosgene, phosphorous tri-bro-mide, phosphorous trichloride, phosphorous pentachloride, phosphorous trichloride oxide or thionyl chloride; anhydride former, such as ethyl chloroformate, methyl chloroformate, isopropyl chloroformate, isobutyl chloroformate or methanesulfonyl chloride; carbodiimides, such as N,N'-dicyclohexylcarbodiimide (DCC) or other customary condensing agents, such as phosphorous pentoxide, polyphosphoric acid, N,N'-carbonyl-diimidazole, 2-ethoxy-N-ethoxycarbonyl-1,2-dihydroquinoline (EEDQ), triphenylphosphine/tetrachloromethane, 4-(4,6-dimethoxy[1.3.5]triazin-2-yl)-4-methylmorpholinium chloride hydrate or bromo-tripyrrolidino-phosphonium-hexafluorophosphate.

Suitable solvents for carrying out processes P1 to P2 according to the invention are customary inert organic solvents. Preference is given to using optionally halogenated aliphatic, alicyclic or aromatic hydrocarbons, such as petroleum ether, hexane, heptane, cyclohexane, methylcyclohexane, benzene, toluene, xylene or decalin ; chlorobenzene, dichlorobenzene, dichloromethane, chloroform, carbon tetrachloride, dichlorethane or trichlorethane ; ethers, such as diethyl ether, diisopropyl ether, methyl tert-butyl ether, methyl tert-amyl ether, dioxane, tetrahydrofuran, 1,2-dimethoxyethane, 1,2-diethoxyethane or anisole ; nitriles, such as acetonitrile, propionitrile, n- or iso-butyronitrile or benzonitrile; amides, such as *N,N*-dimethylformamide, *N,N*-dimethylacetamide, *N*-methylformanilide, *N-*methylpyrrolidone or hexamethylphosphoric triamide ; esters, such as methyl acetate or ethyl acetate, sulphoxides, such as dimethyl sulphoxide, or sulphones, such as sulpholane.

Suitable bases for carrying out processes P1 and P2 according to the invention are inorganic and organic bases which are customary for such reactions. Preference is given to using alkaline earth metal, alkali metal hydride, alkali metal hydroxides or alkali metal alkoxides, such as sodium hydroxide, sodium hydride, calcium hydroxide, potassium hydroxide, potassium tert-butoxide or other ammonium hydroxide, alkali metal carbonates, such as sodium carbonate, potassium carbonate, potassium bicarbonate, sodium bicarbonate, cesium carbonate, alkali metal or alkaline earth metal acetates, such as sodium acetate, potassium acetate, calcium acetate, and also tertiary amines, such as trimethylamine, triethylamine, diisopropylethylamine, tributylamine, *N,N*-dimethylaniline, pyridine, *N*-methylpiperidine, *N*,*N*-dimethylaminopyridine, 1,4-diazabicyclo[2.2.2]octane (DABCO), 1,5-diazabicyclo[4.3.0]non-5-ene (DBN) or 1,8-diazabicyclo[5.4.0]undec-7-ene (DBU).

When carrying out processes P1 to P2 according to the invention, the reaction temperature can independently be varied within a relatively wide range. Generally, processes P1to P2 according to the invention are carried out at temperatures between -80°C and 160°C.

Processes P1 to P2 according to the invention are generally independently carried out under atmospheric pressure. However, it is also possible to operate under elevated or reduced pressure.
Work-up is carried out by customary methods. Generally, the reaction mixture is treated with water and the organic phase is separated off and, after drying, concentrated under reduced pressure. If appropriate, the remaining residue can be freed by customary methods, such as chromatography or recrystallization, from any impurities that may still be present.

Compounds according to the invention can be prepared according to the above described processes. It will nevertheless be understood that, on the basis of his general knowledge and of available publications, the skilled worker will be able to adapt these processes according to the specifics of each of the compounds according to the invention that is desired to be synthesised.

In a further aspect, the present invention also relates to a fungicide composition comprising an effective and non-phytotoxic amount of an active compound of formula (I).

The expression "effective and non-phytotoxic amount" means an amount of composition according to the invention that is sufficient to control or destroy the fungi present or liable to appear on the cropsand that does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials, that are within the capabilities of a person skilled in the art.

Thus, according to the invention, there is provided a fungicide composition comprising, as an active ingredient, an effective amount of a compound of formula (I) as herein-defined and an agriculturally acceptable support, carrier or filler.

According to the invention, the term "support" denotes a natural or synthetic, organic or inorganic compound with that the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports can also be used.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyolsand derivatives of the above compounds containing sulphate, sulphonate and phosphate functions. The presence of at least one surfactant is generally essential when the active compound and/or the inert support are water-insoluble and when the vector agent for the application is water. Preferably, surfactant content can be comprised from 5% to 40% by weight of the composition.

Optionally, additional components can also be included, e.g. protective colloids, adhesives, thickeners, thixotropic agents, penetration agents, stabilisers, sequestering agents. More generally, the active compounds can be combined with any solid or liquid additive, that complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

The compounds according to the invention can also be mixed with one or more insecticide, fungicide, bactericide, attractant, acaricide or pheromone active substance or other compounds with biological activity. The mixtures thus obtained have normally a broadened spectrum of activity. The mixtures with other fungicide compounds are particularly advantageous.

Examples of suitable fungicide mixing partners can be selected in the following lists:
B1) a compound capable to inhibit the nucleic acid synthesis like benalaxyl, benalaxyl-M, bupirimate, clozylacon, dimethirimol, ethirimol, furalaxyl, hymexazol, mefenoxam, metalaxyl, metalaxyl-M, ofurace, oxadixyl, oxolinic acid ;
B2) a compound capable to inhibit the mitosis and cell division like benomyl, carbendazim, diethofencarb, ethaboxam, fuberidazole, pencycuron, thiabendazole, thiophanate-methyl, zoxamide ;
B3) a compound capable to inhibit the respiration for example
   as CI-respiration inhibitor like diflumetorim ;
   as CII-respiration inhibitor like boscalid, carboxin, fenfuram, flutolanil, furametpyr, furmecyclox, mepronil, oxycarboxin, penthiopyrad, thifluzamide ;
   as CIII-respiration inhibitor like amisulbrom, azoxystrobin, cyazofamid, dimoxystrobin, enestrobin, famoxadone, fenamidone, fluoxastrobin, kresoxim-methyl, metominostrobin, orysastrobin, picoxystrobin, pyraclostrobin, trifloxystrobin ;
B4) a compound capable of to act as an uncoupler like dinocap, fluazinam, meptyldinocap ;
B5) a compound capable to inhibit ATP production like fentin acetate, fentin chloride, fentin hydroxide, silthiofam ;
B6) a compound capable to inhibit AA and protein biosynthesis like andoprim, blasticidin-S, cyprodinil, kasugamycin, kasugamycin hydrochloride hydrate, mepanipyrim, pyrimethanil ;
B7) a compound capable to inhibit the signal transduction like fenpiclonil, fludioxonil, quinoxyfen ;
B8) a compound capable to inhibit lipid and membrane synthesis like biphenyl, chlozolinate, edifenphos, etridiazole, iodocarb, iprobenfos, iprodione, isoprothiolane, procymidone, propamocarb, propamocarb hydrochloride, pyrazophos, tolclofos-methyl, vinclozolin ;
B9) a compound capable to inhibit ergosterol biosynthesis like aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, prochloraz, propiconazole, prothioconazole, pyributicarb, pyrifenox, simeconazole, spiroxamine, tebuconazole, terbinafine, tetraconazole, triadimefon, triadimenol, tridemorph, triflumizole, triforine, triticonazole, uniconazole, viniconazole, voriconazole ;
B10) a compound capable to inhibit cell wall synthesis like benthiavalicarb, dimethomorph, flumorph, iprovalicarb, mandipropamid, polyoxins, polyoxorim, validamycin A;
B11) a compound capable to inhibit melanine biosynthesis like carpropamid, diclocymet, fenoxanil, phthalide, pyroquilon, tricyclazole ;
B12) a compound capable to induce a host defence like acibenzolar-S-methyl, probenazole, tiadinil ;
B13) a compound capable to have a multisite action like Bordeaux mixture, captafol, captan, chlorothalonil, copper naphthenate, copper oxide, copper oxychloride, copper preparations such as copper hydroxide, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propineb, sulphur and sulphur preparations including calcium polysulphide, thiram, tolylfluanid, zineb, ziram ;
B14) a compound selected in the following list: 2,3-dibutyl-6-chlorothieno[2,3-d]pyrimidin-4(3H)-one, ethyl (2Z)-3-amino-2-cyano-3-phenylprop-2-enoate, N-[2-(1,3-dimethylbutyl)phenyl]-5-fluoro-1,3-dimethyl-1H-pyrazole-4-carboxamide, N-{2-[1,1'-bi(cyclopropyl)-2-yl]phenyl}-3-(difluoromethyl)-1-methyl-1H-pyrazole-4-carboxamide, 3-(difluoromethyl)-1-methyl-N-(3',4',5'-trifluorobiphenyl-2-yl)-1H-pyrazole-4-carboxamide, 3-(difluoromethyl)-N-[4-fluoro-2-(1,1,2,3,3,3-hexafluoropropoxy)phenyl]-1-methyl-1H-pyrazole-4-carboxamide, (2E)-2-(2-{[6-(3-chloro-2-methylphenoxy)-5-fluoropyrimidin-4-yl]oxy}phenyl)-2-(methoxyimino)-N-methylethanamide, (2E)-2-{2-[({[(2E,3E)-4-(2,6-dichlorophenyl)but-3-en-2-ylidene]amino}oxy)methyl]phenyl}-2-(methoxyimino)-N-methylethanamide, 2-chloro-N-(1,1,3-trimethyl-2,3-dihydro-1H-inden-4-yl)pyridine-3-carboxamide, N-(3-ethyl-3,5,5-trimethylcyclohexyl)-3-(formylamino)-2-hydroxy benzamide, 5-methoxy-2-methyl-4-(2-{[({(1E)-1-[3-(trifluoromethyl)phenyl]ethylidene}amino)oxy] methyl}phenyl)-2,4-dihydro-3H-1,2,4-triazol-3-one, (2E)-2-(methoxyimino)-N-methyl-2-(2-{[({(1E)-1-[3-(trifluoromethyl)phenyl]ethylidene}amino)oxy] methyl}phenyl)ethanamide, (2E)-2-(methoxyimino)-N-methyl-2-{2-[(E)-({1-[3-(trifluoromethyl)phenyl]ethoxy}imino)methyl]phenyl} ethanamide, (2E)-2-{2-[({[(1E)-1-(3-{[(E)-1-fluoro-2-phenylethenyl]oxy}phenyl)ethylidene]amino} oxy)methyl]phenyl}-2-(methoxyimino)-N-methylethanamide, 1-(4-chlorophenyl)-2-(1H-1,2,4-triazol-1-yl)cycloheptanol, methyl 1-(2,2-dimethyl-2,3-dihydro-1H-inden-1-yl)-1H-imidazole-5-carboxylate, N-ethyl-N-methyl-N'-{2-methyl-5-(trifluoromethyl)-4-[3-(trimethylsilyl)propoxy] phenyl}imidoformamide, N'-{5-(difluoromethyl)-2-methyl-4-[3-(trimethylsilyl)propoxy]phenyl}-N-ethyl-N-methylimidoformamide, O-{1-[(4-methoxyphenoxy)methyl]-2,2-dimethylpropyl} 1H-imidazole-1-carbothioate, N-[2-(4-{[3-(4-chlorophenyl)prop-2-yn-1-yl]oxy}-3-methoxyphenyl) ethyl]-N²-(methylsulfonyl)valinamide, 5-chloro-7-(4-methylpiperidin-1-yl)-6-(2,4,6-trifluorophenyl) [1,2,4]triazolo[1,5-a]pyrimidine, 5-amino-1,3,4-thiadiazole-2-thiol, propamocarb-fosetyl, 1-[(4-methoxyphenoxy)methyl]-2,2-dimethylpropyl 1H-imidazole-1-carboxylate, 1-methyl-N-[2-(1,1,2,2-tetrafluoroethoxy)phenyl]-3-(trifluoromethyl)-1H-pyrazole-4-carboxamide, 2,3,5,6-tetrachloro-4-(methylsulfonyl)pyridine, 2-butoxy-6-iodo-3-propyl-4H-chromen-4-one, 2-phenylphenol and salts, 3-(difluoromethyl)-1-methyl-N-[2-(1,1,2,2-tetrafluoroethoxy)phenyl]-1H-pyrazole-4-carboxamide, 3,4,5-trichloropyridine-2,6-dicarbonitrile, 3-[5-(4-chlorophenyl)-2,3-dimethylisoxazolidin-3-yl]pyridine, 3-chloro-5-(4-chlorophenyl)-4-(2,6-difluorophenyl)-6-methylpyridazine, 4-(4-chlorophenyl)-5-(2,6-difluorophenyl)-3,6-dimethylpyridazine, quinolin-8-ol, quinolin-8-ol sulfate (2:1) (salt), 5-methyl-6-octyl-3,7-dihydro[1,2,4]triazolo[1,5-a]pyrimidin-7-amine, 5-ethyl-6-octyl-3,7-dihydro[1,2,4]triazolo[1,5-a]pyrimidin-7-amine, benthiazole, bethoxazin, capsimycin, carvone, chinomethionat, chloroneb, cufraneb, cyflufenamid, cymoxanil, cyprosulfamide, dazomet, debacarb, dichlorophen, diclomezine, dicloran, difenzoquat, difenzoquat methylsulphate, diphenylamine, ecomate, ferimzone, flumetover, fluopicolide, fluoroimide, flusulfamide, fosetyl-aluminium, fosetyl-calcium, fosetyl-sodium, hexachlorobenzene, irumamycin, isotianil, methasulfocarb, methyl (2E)-2-{2-[({cyclopropyl[(4-methoxyphenyl)imino]methyl}thio)methyl]phenyl}-3-methoxyacrylate, methyl isothiocyanate, metrafenone, (5-bromo-2-methoxy-4-methylpyridin-3-yl)(2,3,4-trimethoxy-6-methylphenyl) methanone, mildiomycin, tolnifanide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloropyridine-3-carboxamide, N-[1-(5-bromo-3-chloropyridin-2-yl)ethyl]-2,4-dichloropyridine-3-carboxamide, N-[1-(5-bromo-3-chloropyridin-2-yl)ethyl]-2-fluoro-4-iodopyridine-3-carboxamide, N-{(Z)-[(cyclopropylmethoxy)imino][6-(difluoromethoxy)-2,3-difluorophenyl]methyl}-2-phenylacetamide, N-{(E)-[(cyclopropylmethoxy)imino][6-(difluoromethoxy)-2,3-difluorophenyl]methyl}-2-phenylacetamide, natamycin, nickel dimethyldithiocarbamate, nitrothal-isopropyl, octhilinone, oxamocarb, oxyfenthiin, pentachlorophenol and salts, phenazine-1-carboxylic acid, phenothrin, phosphorous acid and its salts, propamocarb fosetylate, propanosine-sodium, proquinazid, pyrrolnitrine, quintozene, S-prop-2-en-1-yl 5-amino-2-(1-methylethyl)-4-(2-methylphenyl)-3-oxo-2,3-dihydro-1H-pyrazole-1-carbothioate, tecloftalam, tecnazene, triazoxide, trichlamide, 5-chloro-N'-phenyl-N'-prop-2-yn-1-ylthiophene-2-sulfonohydrazide and zarilamid.

The composition according to the invention comprising a mixture of a compound of formula
(I) with a bactericide compound can also be particularly advantageous. Examples of suitable bactericide mixing partners can be selected in the following list: bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.

The compounds of formula (I) and the fungicide composition according to the invention can be used to curatively or preventively control the phytopathogenic fungi of plants or crops.
Thus, according to a further aspect of the invention, there is provided a method for curatively or preventively controlling the phytopathogenic fungi of plants or crops characterised in that a compound of formula (I) or a fungicide composition according to the invention is applied to the seed, the plant or to the fruit of the plant or to the soil wherein the plant is growing or wherein it is desired to grow.
The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the overground parts of the plant such as trunks, stems or stalks, leaves, flowers and fruit of the concerned plant.

Among the plants that can be protected by the method according to the invention, mention can be made of cotton; flax; vine; fruit or vegetable crops such as *Rosaceae sp.* (for instance pip fruit such as apples and pears, but also stone fruit such as apricots, almonds and peaches), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantins), *Rubiaceae sp., Theaceae sp., Sterculiceae sp., Rutaceae sp.* (for instance lemons oranges and grapefruit); *Solanaceae sp.* (for instance tomatoes), *Liliaceae sp., Asteraceae sp.* (for instance lettuces), *Umbelliferae sp., Cruciferae sp., Chenopodiaceae sp., Cucurbitaceae sp., Papilionaceae sp.* (for instance peas), *Rosaceae sp.* (for instance strawberries); major crops such as *Graminae sp.* (for instance maize, lawn or cereals such as wheat, rye, rice, barley and triticale), *Asteraceae sp.* (for instance sunflower), *Cruciferae sp.* (for instance colza), *Fabacae sp.* (for instance peanuts), *Papilionaceae sp.* (for instance soybean), *Solanaceae sp.* (for instance potatoes), *Chenopodiaceae sp.* (for instance beetroots), *Elaeis sp.* (for instance oil palm); horticultural and forest crops; as well as genetically modified homologues of these crops.

The composition according to the invention may also be used in the treatment of genetically modified organisms with the compounds according to the invention or the agrochemical compositions according to the invention. Genetically modified plants are plants into genome of which a heterologous gene encoding a protein of interest has been stably integrated. The expression "heterologous gene encoding a protein of interest" essentially means genes which give the transformed plant new agronomic properties or genes for improving the agronomic quality of the modified plant.

The composition according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention or a composition according to the invention; this includes for example direct application, spraying, dipping, injection or any other suitable means.

Among the diseases of plants or crops that can be controlled by the method according to the invention, mention can be made of :
- Powdery mildew diseases such as :
   Blumeria diseases, caused for example by *Blumeria graminis*;
   Podosphaera diseases, caused for example by *Podosphaera leucotricha*;
   Sphaerotheca diseases, caused for example by *Sphaerotheca fuliginea*;
   Uncinula diseases, caused for example by *Uncinula necator*;
      • Rust diseases such as :

   Gymnosporangium diseases, caused for example by *Gymnosporangium sabinae*;
   Hemileia diseases, caused for example by *Hemileia vastatrix*;
   Phakopsora diseases, caused for example by *Phakopsora pachyrhizi* or *Phakopsora meibomiae*;
   Puccinia diseases, caused for example by *Puccinia recondita*;
   Uromyces diseases, caused for example by *Uromyces appendiculatus*;
      - Oomycete diseases such as :

   Albugo diseases caused for example by *Albugo candida*;
   Bremia diseases, caused for example by *Bremia lactucae*;
   Peronospora diseases, caused for example by *Peronospora pisi* or *P. brassicae*;
   Phytophthora diseases, caused for example by *Phytophthora infestans* ;
   Plasmopara diseases, caused for example by *Plasmopara viticola*;
   Pseudoperonospora diseases, caused for example by *Pseudoperonospora humuli* or *Pseudoperonospora cubensis*;
   Pythium diseases, caused for example by *Pythium ultimum*;
      - Leafspot, leaf blotch and leaf blight diseases such as:

   Alternaria diseases, caused for example by *Alternaria solani*;
   Cercospora diseases, caused for example by *Cercospora beticola* ;
   Cladiosporum diseases, caused for example by *Cladiosporium cucumerinum* ;
   Cochliobolus diseases, caused for example by *Cochliobolus sativus* ;
   Colletotrichum diseases, caused for example by *Colletotrichum lindemuthanium* ;
   Cycloconium diseases, caused for example by *Cycloconium oleaginum* ;
   Diaporthe diseases, caused for example by *Diaporthe citri* ;
   Drechslera, Syn: Helminthosporium) or *Cochliobolus miyabeanus* ;
   Elsinoe diseases, caused for example by *Elsinoe fawcettii* ;
   Gloeosporium diseases, caused for example by *Gloeosporium laeticolor* ;
   Glomerella diseases, caused for example by *Glomerella cingulata* ;
   Guignardia diseases, caused for example by *Guignardia bidwelli* ;
   Leptosphaeria diseases, caused for example by *Leptosphaeria maculans*; *Leptosphaeria nodorum* ;
   Magnaporthe diseases, caused for example by *Magnaporthe grisea* ;
   Mycosphaerella diseases, caused for example by *Mycosphaerella graminicola* ; *Mycosphaerella arachidicola*; *Mycosphaerella fijiensis* ;
   Phaeosphaeria diseases, caused for example by *Phaeosphaeria nodorum* ;
   Pyrenophora diseases, caused for example by *Pyrenophora teres* ;
   Ramularia diseases, caused for example by *Ramularia collo-cygni* ;
   Rhynchosporium diseases, caused for example by *Rhynchosporium secalis* ;
   Septoria diseases, caused for example by *Septoria apii* or *Septoria lycopercisi* ;
   Typhula diseases, caused for example by *Typhula incarnata* ;
   Venturia diseases, caused for example by *Venturia inaequalis* ;
      - Root and stem diseases such as :

   Corticium diseases, caused for example by *Corticium graminearum* ;
   Fusarium diseases, caused for example by *Fusarium oxysporum* ;
   Gaeumannomyces diseases, caused for example by *Gaeumannomyces graminis* ;
   Rhizoctonia diseases, caused for example by *Rhizoctonia solani* ;
   Sarocladium diseases caused for example by *Sarocladium oryzae;*
   Sclerotium diseases caused for example by *Sclerotium oryzae*;
   Tapesia diseases, caused for example by *Tapesia acuformis* ;
   Thielaviopsis diseases, caused for example by *Thielaviopsis basicola* ;
      - Ear and panicle diseases including maize cob, such as :

   Alternaria diseases, caused for example by *Alternaria spp.* ;
   Aspergillus diseases, caused for example by *Aspergillus flavus* ;
   Cladosporium diseases, caused for example by *Cladosporium spp*. ;
   Claviceps diseases, caused for example by *Claviceps purpurea* ;
   Fusarium diseases, caused for example by *Fusarium culmorum* ;
   Gibberella diseases, caused for example by *Gibberella zeae* ;
   Monographella diseases, caused for example by *Monographella nivalis* ;
      - Smut and bunt diseases such as :

   Sphacelotheca diseases, caused for example by *Sphacelotheca reiliana* ;
   Tilletia diseases, caused for example by *Tilletia caries* ;
   Urocystis diseases, caused for example by *Urocystis occulta* ;
   Ustilago diseases, caused for example by *Ustilago nuda* ;
      - Fruit rot and mould diseases such as :

   Aspergillus diseases, caused for example by *Aspergillus flavus* ;
   Botrytis diseases, caused for example by *Botrytis cinerea* ;
   Penicillium diseases, caused for example by *Penicillium expansum* ;
   Rhizopus diseases caused by example by *Rhizopus stolonifer*
   Sclerotinia diseases, caused for example by *Sclerotinia sclerofiorum* ;
   Verticilium diseases, caused for example by *Verticilium alboatrum* ;
      - Seed and soil borne decay, mould, wilt, rot and damping-off diseases such as :

   Alternaria diseases, caused for example by *Alternaria brassicicola*
   Aphanomyces diseases, caused for example by *Aphanomyces euteiches*
   Ascochyta diseases, caused for example by *Ascochyta lentis*
   Aspergillus diseases, caused for example by *Aspergillus flavus*
   Cladosporium diseases, caused for example by *Cladosporium herbarum*
   Cochliobolus diseases, caused for example by *Cochliobolus sativus*
   (Conidiaform: *Drechslera, Bipolaris Syn: Helminthosporium*);
   Colletotrichum diseases, caused for example by *Colletotrichum coccodes*;
   Fusarium diseases, caused for example by *Fusarium culmorum*;
   Gibberella diseases, caused for example by *Gibberella zeae*;
   Macrophomina diseases, caused for example by *Macrophomina* phaseolina
   Monographella diseases, caused for example by *Monographella nivalis*;
   Penicillium diseases, caused for example by *Penicillium expansum*
   Phoma diseases, caused for example by *Phoma lingam*
   Phomopsis diseases, caused for example by *Phomopsis sojae*;
   Phytophthora diseases, caused for example by Phytophthora cactorum;
   Pyrenophora diseases, caused for example by *Pyrenophora graminea*
   Pyricularia diseases, caused for example by *Pyricularia oryzae*;
   Pythium diseases, caused for example by *Pythium ultimum*;
   Rhizoctonia diseases, caused for example by *Rhizoctonia solani*;
   Rhizopus diseases, caused for example by *Rhizopus oryzae*
   Sclerotium diseases, caused for example by *Sclerotium rolfsii*;
   Septoria diseases, caused for example by *Septoria nodorum*;
   Typhula diseases, caused for example by *Typhula incarnata*;
   Verticillium diseases, caused for example by *Verticillium dahliae* ;
      - Canker, broom and dieback diseases such as :
   Nectria diseases, caused for example by *Nectria galligena* ;
      - Blight diseases such as :

   Monilinia diseases, caused for example by *Monilinia laxa* ;
      - Leaf blister or leaf curl diseases such as :

   Exobasidium diseases caused for nexample by *Exobasidium vexans;*
   Taphrina diseases, caused for example by *Taphrina deformans* ;
      - Decline diseases of wooden plants such as :

   Esca diseases, caused for example by *Phaemoniella clamydospora, Phaeomoniella*
   *clamydospora, Phaeoacremonium aleophilum and Fomitiporia mediterranea* ;
   Eutypa dyeback, caused for example by *Eutypa lata* ;
   Dutch elm disease, caused for example by *Ceratocystsc ulmi* ;
   Ganoderma diseases caused by example by Ganoderma boninense;
      - Diseases of flowers and Seeds such as :

   Botrytis diseases, caused for example by *Botrytis cinerea* ;
      - Diseases of tubers such as :

   Rhizoctonia diseases, caused for example by *Rhizoctonia solani*
   Helminthosporium diseases, caused for example by *Helminthosporium solani.*
      - Diseases of Tubers such as

   Rhizoctonia diseases caused for example by *Rhizoctonia solani* ;
   Helminthosporium diseases caused for example by *Helminthosporium solani* ;
      • Club root diseases such as
   Plasmodiophora diseases, caused for example by *Plamodiophora brassicae* ;
      • Diseases caused by Bacterial Organisms such as
   Xanthomanas species for example *Xanthomonas campestris pv. oryzae*;
   Pseudomonas species for example *Pseudomonas syringae pv. lachrymans*;
   Erwinia species for example *Erwinia amylovora.*

The fungicide composition according to the invention can also be used against fungal diseases liable to grow on or inside timber. The term "timber" means all types of species of woodand all types of working of this wood intended for construction, for example solid wood, high-density wood, laminated wood and plywood. The method for treating timber according to the invention mainly consists in contacting one or more compounds according to the invention, or a composition according to the invention ; this includes for example direct application, spraying, dipping, injection or any other suitable means.

The dose of active compound usually applied in the method of treatment according to the invention is generally and advantageously from 10 to 800 g/ha, preferably from 50 to 300 g/ha for applications in foliar treatment. The dose of active substance applied is generally and advantageously from 2 to 200 g per 100 kg of seed, preferably from 3 to 150 g per 100 kg of seed in the case of seed treatment.
It is clearly understood that the doses indicated herein are given as illustrative examples of the method according to the invention. A person skilled in the art will know how to adapt the application doses, notably according to the nature of the plant or crop to be treated.

The fungicide composition according to the invention can also be used in the treatment of genetically modified organisms with the compounds according to the invention or the agrochemical compositions according to the invention. Genetically modified plants are plants into genome of that a heterologous gene encoding a protein of interest has been stably integrated. The expression "heterologous gene encoding a protein of interest" essentially means genes that give the transformed plant new agronomic properties, or genes for improving the agronomic quality of the modified plant.

The compounds or mixtures according to the invention can also be used for the preparation of composition useful to curatively or preventively treat human or animal fungal diseases such as, for example, mycoses, dermatoses, trichophyton diseases and candidiases or diseases caused by *Aspergillus spp.,* for example *Aspergillus fumigatus.*

## Claims

1. A compound of formula (I) 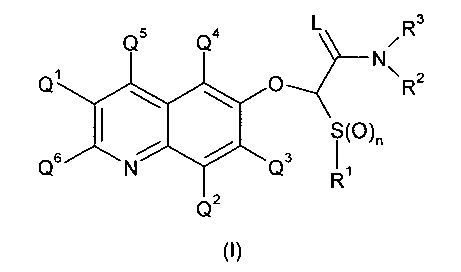 wherein
• Q¹ and Q² independently represent a hydrogen atom, a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶-sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
• Q³ represents a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-□⁶-sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
• Q⁴, Q⁵ and Q⁶ independently represent a hydrogen atom, a halogen atom, a nitro group, a hydroxy group, a cyano group, an amino group, a sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a pentafluoro-λ⁶-sulphenyl group, substituted or non-substituted C₁-C₈-alkoxyamino group, substituted or non-substituted N-C₁-C₈-alkyl-(C₁-C₈-alkoxy)-amino group, substituted or non-substituted (C₁-C₈-alkylamino)-amino group, substituted or non-substituted N-C₁-C₈-alkyl-(C₁-C₈-alkylamino)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkylamino, substituted or non-substituted di-C₁-C₈-alkylamino, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₁-C₈-halogenoalkylsulphenyl having 1 to 5 halogen atoms, substituted or non-substituted C₂-C₈-alkenyloxy, substituted or non-substituted C₂-C₈-halogenoalkenyloxy having 1 to 5 halogen atoms, substituted or non-substituted C₃-C₈-alkynyloxy, substituted or non-substituted C₃-C₈-halogenoalkynyloxy having 1 to 5 halogen atoms, substituted or non-substituted C₁-C₈-alkylcarbonyl, substituted or non-substituted C₁-C₈-halogenoalkylcarbonyl having 1 to 5 halogen atoms, substituted or non-substituted C₁-C₈-alkylcarbamoyl, substituted or non-substituted di-C₁-C₈-alkylcarbamoyl, substituted or non-substituted N-C₁-C₈-alkyloxycarbamoyl, substituted or non-substituted C₁-C₈-alkoxycarbamoyl, substituted or non-substituted N-C₁-C₈-alkyl-C₁-C₈-alkoxycarbamoyl, substituted or non-substituted C₁-C₈-alkoxycarbonyl, substituted or non-substituted C₁-C₈-halogenoalkoxycarbonyl having 1 to 5 halogen atoms, substituted or non-substituted C₁-C₈-alkylcarbonyloxy, substituted or non-substituted C₁-C₈-halogenoalkylcarbonyloxy having 1 to 5 halogen atoms, substituted or non-substituted C₁-C₈-alkylcarbonylamino, substituted or non-substituted C₁-C₈-halogenoalkylcarbonylamino having 1 to 5 halogen atoms, substituted or non-substituted C₁-C₈-alkylcarbamoylamino, substituted or non-substituted C₁-C₈-halogenoalkylcarbamoylamino having 1 to 5 halogen atoms, substituted or non-substituted di-C₁-C₈-alkylcarbamoylamino, substituted or non-substituted di-C₁-C₈-halogenoalkylcarbamoylamino having 1 to 5 halogen atoms, substituted or non-substituted N-C₁-C₈-alkyl-(C₁-C₈-alkylcarbamoyl)amino, substituted or non-substituted N-C₁-C₈-alkyl-(C₁-C₈-halogenoalkylcarbamoyl)amino having 1 to 5 halogen atoms, substituted or non-substituted N-C₁-C₈-alkyl-(di-C₁-C₈-alkylcarbamoyl)amino, substituted or non-substituted N-C₁-C₈-alkyl-(di-C₁-C₈-halogenoalkylcarbamoyl)amino having 1 to 5 halogen atoms, substituted or non-substituted C₁-C₈-alkylaminocarbonyloxy, substituted or non-substituted di-C₁-C₈-alkylaminocarbonyloxy, substituted or non-substituted C₁-C₈-alkylcarbamothioyl, substituted or non-substituted di-C₁-C₈-alkylcarbamothioyl, substituted or non-substituted N-C₁-C₈-alkyloxycarbamothioyl, substituted or non-substituted C₁-C₈-alkoxycarbamothioyl, substituted or non-substituted N-C₁-C₈-alkyl-C₁-C₈-alkoxycarbamothioyl, substituted or non-substituted C₁-C₈-alkylthioylamino, substituted or non-substituted C₁-C₈-halogenoalkylthioylamino having 1 to 5 halogen atoms, substituted or non-substituted (C₁-C₈-alkyl-carbamothioyl)-oxy, substituted or non-substituted substituted or non-substituted (di-C₁-C₈-alkyl-carbamothioyl)-oxy, substituted or non-substituted C₁-C₈-alkylsulphinyl, substituted or non-substituted C₁-C₈-halogenoalkylsulphinyl having 1 to 5 halogen atoms, substituted or non-substituted C₁-C₈-alkylsulphonyl, substituted or non-substituted C₁-C₈-halogenoalkylsulphonyl having 1 to 5 halogen atoms, substituted or non-substituted C₁-C₈-alkylaminosulfamoyl, substituted or non-substituted di-C₁-C₈-alkylaminosulfamoyl, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted benzylamino, substituted or non-substituted phenoxy, substituted or non-substituted phenylsulphenyl, substituted or non-substituted phenylamino, substituted or non-substituted aryl, substituted or non-substituted aryl-[C₁-C₈]-alkyl, substituted or non-substituted tri(C₁-C₈-alkyl)-silyloxy, substituted or non-substituted C₁-C₈-alkylsulfenylamino, substituted or non-substituted C₁-C₈-halogenoalkylsulphinylamino having 1 to 5 halogen atoms, substituted or non-substituted C₁-C₈-alkylsulphonylamino, substituted or non-substituted C₁-C₈-halogenoalkylsulphonylamino having 1 to 5 halogen atoms, substituted or non-substituted C₁-C₈-alkoxysulphonylamino, substituted or non-substituted C₁-C₈-halogenoxysulphonylamino having 1 to 5 halogen atoms, substituted or non-substituted (C₁-C₆-alkylideneamino)oxy, substituted or non-substituted (C₁-C₆-alkenylideneamino)oxy, substituted or non-substituted (C₁-C₆-alkynylideneamino)oxy, substituted or non-substituted (benzylideneamino)oxy; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S; provided that at least one of Q⁴, Q⁵ or Q⁶ does not represent a hydrogen atom;
• R¹ represents a hydrogen atom, substituted or non-substituted C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted aryl, substituted or non-substituted aryl-[C₁-C₈]-alkyl, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S;
• R² represents a hydrogen atom, a hydroxy group, a cyano group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkoxy, substituted or non-substituted benzyloxy:
• R³ represents -(CR^{a}R^{b})ₚ(CR^{c}R^{d})_{q}(X)ᵣ(CR^{e}R^{f})ₛR⁴, wherein o R^{a}, R^{b}, R^{c}, R^{d}, R^{e} and R^{f}, independently represent a hydrogen atom, substituted or non-substituted C₁-C₈-alkyl, a halogen atom, a cyano group, a hydroxy group, substituted or non-substituted C₁-C₈-alkoxy or substituted or non-substituted C₁-C₈-alkoxycarbonyl, or
o R^{a}R^{b}, R^{c}R^{d} or R^{e}R^{f} form a 3- to 8-membered carbocyclic or heterocyclic ring comprising a heteroatom selected from sulfur, oxygen and NR⁰ wherein R⁰ represents a hydrogen atom or substituted or non-substituted C₁-C₈-alkyl;
o X represents (CO), (CO)O, O(CO), O, S(O)ₜ, wherein t is 0, 1 or 2; or X represents NH, substituted or non-substituted C₁-C₈-alkylamino ;
o p, r and s, independently represent 0 or 1 ;
o q represents 0, 1 or 2 ;
o R⁴ represents a halogen atom, substituted or non-substituted C₁-C₈-alkyl, substituted or non-substituted C₁-C₈-alkenyl, -CR^{uu}=NR^{w} wherein R^{uu} represents a hydrogen atom or substituted or non-substituted C₁-C₈-alkyl and R^{w} represents a hydroxy group or substituted or non-substituted C₁-C₈-alkoxy, substituted or non-substituted aryloxy or substituted or non-substituted heteroaryloxy or R⁴ represents CH₂-C≡C-R⁵ wherein R⁵ represents a hydrogen atom, substituted or non-substituted C₁-C₈-alkyl, substituted or non-substituted C₃-C₈-cycloalkyl, substituted or non-substituted aryl, substituted or non-substituted aryl-[C₁-C₈]-alkyl, substituted or non-substituted tri(C₁-C₈-alkyl)-silyl; substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S, or R⁴ represents substituted or non-substituted C₃-C₈-cycloalkyl, substituted or non-substituted C₃-C₈-cycloalkenyl, substituted or non-substituted aryl, saturated or unsaturated 4-, 5-, 6- or 7-membered heterocyclyl comprising up to 4 heteroatoms selected in the list consisting of N, O, S; or
• when R³ represents -(CR^{a}R^{b})ₚ(CR^{c}R^{d})_{q}(X)ᵣ(CR^{e}R^{f})ₛR⁴, R² and R³ form a 5- or 6-membered ring optionally substituted with a halogen atom, C₁-C₈-alkyl, mono- or di-C₁-C₈-alkylaminocarbonyl, and optionally containing a heteroatom selected from sulphur, oxygen and NR⁰⁰, wherein R⁰⁰ represents C₁-C₈-alkyl, C₁-C₈-halogenoalkyl having 1 to 5 halogen atoms, C₁-C₈-alkoxy, cyano, substituted or non-substituted aryl; or
• R² and R³ form an optionally substituted 6,6-membered bicycle; or
• R³ represents -(CR³⁰R⁴⁰)C≡CR⁵⁰, wherein o R³⁰ and R⁴⁰ independently represent a hydrogen atom, substituted or non-substituted C₁-C₈-alkyl, substituted or non-substituted C₁-C₈-halogenoalkyl having 1 to 5 halogen atoms, a C₂-C₈-alkenyl, substituted or non-substituted C₂-C₈-alkynyl, or o R³⁰ and R⁴⁰ join with the carbon atom to which they are attached to form a substituted or non-substituted 3- to 6-membered carbocyclic or heterocyclic ring containing a heteroatom selected from sulfur, oxygen or NR⁰⁰⁰ wherein R⁰⁰⁰ represents a hydrogen atom, C₁-C₈-alkyl; o R⁵⁰ represents a hydrogen atom, substituted or non-substituted C₁-C₈-alkyl, substituted or non-substituted C₃-C₈-cycloalkyl" substituted or non-substituted aryl, saturated or unsaturated 4-, 5-, 6- or 7-membered heterocyclyl comprising up to 4 heteroatoms selected in the list consisting of N, O, S,
• n is 0, 1 or 2 ;
• L represents a sulfur atom or an oxygen atom ; as well as salts, N-oxides, metallic complexes and metalloidic complexes thereof.

2. A compound according to claim 1 wherein Q¹ and Q² independently represent a hydrogen atom, a halogen atom, a cyano group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₃-C₈-alkynyloxy, substituted or non-substituted phenoxy, substituted or non-substituted aryl, substituted or non-substituted aryl-[C₁-C₈]-alkyl, substituted or non-substituted tri(C₁-C₈-alkyl)-silyloxy, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S.

3. A compound according to claim 2 wherein Q¹ and Q² independently represent a hydrogen atom, a halogen atom, substituted or non-substituted C₁-C₈-alkyl.

4. A compound according to claims 1 to 3 wherein Q³ represents a halogen atom, a cyano group, substituted or non-substituted C₁-C₈-alkyl, substituted or non-substituted tri(C₁-C₈-alkyl)silyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy.

5. A compound according to claim 4 wherein Q³ represents a halogen atom, substituted or non-substituted C₁-C₈-alkyl.

6. A compound according to claims 1 to 5 wherein Q⁴, Q⁵ and Q⁶ independently represent a hydrogen atom, a halogen atom, a cyano group, substituted or non-substituted C₁-C₈-alkyl, substituted or non-substituted tri(C1-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted C₁-C₈-halogenoalkyl having 1 to 5 halogen atoms, substituted or non-substituted C₁-C₈-alkoxy, substituted or non-substituted C₁-C₈-halogenoalkoxy having 1 to 5 halogen atoms, substituted or non-substituted C₁-C₈-alkylsulphenyl, substituted or non-substituted C₃-C₈-alkynyloxy, substituted or non-substituted phenoxy, substituted or non-substituted aryl, substituted or non-substituted aryl-[C₁-C₈]-alkyl, substituted or non-substituted tri(C₁-C₈-alkyl)-silyloxy, substituted or non-substituted, saturated or unsaturated 4-, 5-, 6- or 7-membered heterocyclyl comprising up to 4 heteroatoms selected in the list consisting of N, O, S; substituted or non-substituted, saturated or unsaturated 4-, 5-, 6- or 7-membered heterocyclyl-[C₁-C₈]-alkyl comprising up to 4 heteroatoms selected in the list consisting of N, O, S.

7. A compound according to claims 1 to 6 wherein R¹ represents a hydrogen atom, a hydroxy group, a substituted or non-substituted carbaldehyde O-(C₁-C₈-alkyl)oxime, a formyloxy group, substituted or non-substituted C₁-C₈-alkoxyamino group, substituted or non-substituted N-C₁-C₈-alkyl-(C₁-C₈-alkoxy)-amino group, a substituted or non-substituted (hydroxyimino)-C₁-C₆-alkyl group, substituted or non-substituted C₁-C₈-alkyl, substituted or non-substituted tri(C₁-C₈-alkyl)silyl, substituted or non-substituted tri(C₁-C₈-alkyl)silyl-C₁-C₈-alkyl, substituted or non-substituted C₁-C₈-cycloalkyl, substituted or non-substituted tri(C₁-C₈-alkyl)silyl-C₁-C₈-cycloalkyl, substituted or non-substituted C₁-C₈-halogenoalkyl having 1 to 5 halogen atoms, substituted or non-substituted C₁-C₈-halogenocycloalkyl having 1 to 5 halogen atoms, a C₂-C₈-alkenyl, substituted or non-substituted C₂-C₈-alkynyl, substituted or non-substituted C₁-C₈-alkoxy, substituted or non-substituted C₁-C₈-halogenoalkoxy having 1 to 5 halogen atoms, substituted or non-substituted C₂-C₈-alkenyloxy, substituted or non-substituted C₃-C₈-alkynyloxy, substituted or non-substituted C₁-C₈-alkylcarbonyloxy, substituted or non-substituted (C₁-C₆-alkoxyimino)-C₁-C₆-alkyl, substituted or non-substituted (C₁-C₆-alkenyloxyimino)-C₁-C₆-alkyl, substituted or non-substituted (C₁-C₆-alkynyloxyimino)-C₁-C₆-alkyl, substituted or non-substituted (benzyloxyimino)-C₁-C₆-alkyl, substituted or non-substituted benzyloxy, substituted or non-substituted benzylsulphenyl, substituted or non-substituted phenoxy, substituted or non-substituted phenylsulphenyl, substituted or non-substituted aryl, substituted or non-substituted aryl-[C₁-C₈]-alkyl, substituted or non-substituted tri(C₁-C₈-alkyl)-silyloxy.

8. A compound according to claim 7 wherein R¹ represents a substituted or non-substituted C₁-C₈-alkyl, substituted or non-substituted C₃-C₈-cycloalkyl, substituted or non-substituted C₁-C₈-halogenoalkyl having 1 to 5 halogen atoms, a C₂-C₈-alkenyl, substituted or non-substituted C₂-C₈-alkynyl, substituted or non-substituted aryl, substituted or non-substituted aryl-[C₁-C₈]-alkyl.

9. A compound according to claim 8 wherein R¹ represents a substituted or non-substituted C₁-C₈-alkyl, substituted or non-substituted C₃-C₈-cycloalkyl, a C₂-C₈-alkenyl, substituted or non-substituted C₂-C₈-alkynyl.

10. A compound according to claims 1 to 9 wherein R² represents a hydrogen atom, substituted or non-substituted C₁-C₈-alkyl, substituted or non-substituted C₁-C₈-cycloalkyl.

11. A compound according to claims 1 to 10 wherein L represents an oxygen atom.

12. A fungicide composition comprising, as an active ingredient, an effective amount of a compound of formula (I) according to claims 1 to 11 and an agriculturally acceptable support, carrier or filler.

13. A method for controlling phytopathogenic fungi of crops, **characterized in that** an agronomically effective and substantially non-phytotoxic quantity of a compound according to claims 1 to 11 or a composition according to claim 12 is applied to the soil where plants grow or are capable of growing, to the leaves or the fruit of plants or to the seeds of plants.
